# EUROPEAN PATENT APPLICATION

(11) **EP 1 745 705 A1**
(43) Date of publication of application: **24.01.2007**
(21) Application number: 05015777.5
(22) Date of filing: 20.07.2005
(51) Int. Cl.: A23L 1/308, A23L 1/00, A23L 1/0524, A61K 31/702, A61K 31/732, C08B 37/00

(54) **Process for preparing uronic acid oligosaccharides by extrusion**

(71) Applicant: N.V. Nutricia, 2712 HM Zoetermeer (NL)
(72) Inventor: Stahl, Bernd , Dr., 61191 Rosbach (DE); van Baalen, Ton, A. Dr., 6843 RC Arnhem (NL)
(74) Representative: Köster, Hajo

(57) **Abstract**

The present invention provides processes for the manufacture of uronic acid oligosaccharides using extrusion. The uronic acid polymers are extruded with an enzyme, under basic conditions (pH>9) and/or under high shear.

## Description

### FIELD OF THE INVENTION

The present invention relates to a process for the production of uronic acid oligosaccharides by extrusion.

### BACKGROUND OF THE INVENTION

Degradation products of viscous, soluble, polymeric dietary fibers are described to have beneficial effects when administered to for instance human beings in the form of a dietetical or pharmaceutical composition. For example these degradation products prevent adherence of pathogenic microorganisms to the epithelium of the intestinal tract and prevent infections and/or diarrhea. Furthermore this interference with cell-cell interactions is also effective in the development and maintenance of a healthy immunsystem to avoid diseases like allergiesand cancer. These degradation products can be obtained by an enzymatic breakdown of uronic acid polymers.

WO 02/42484 discloses a method for producing pectin hydrolysis products, the thus obtained pectin hydrolysis products, and the use of said products.

EP0432210 discloses a method to treat a starting product rich in broken-down plant cell walls. Water is added, if necessary, to obtain a mixture suitable for the subsequent shearing treatment. The product is subjected to a shearing stress before extrusion, to obtain a modified product containing a water-soluble polysaccharide fraction greater than that which exists in nature, without changing the overall chemical composition. The modified product is obtained in the form of crushable aggregates.

### SUMMARY OF THE INVENTION

It has surprisingly been found that uronic acid oligosaccharides can advantageously be produced by extruding uronic acid polymers (e.g. pectin) in the presence of at least one enzyme degrading uronic acid polymers (hereinafter also referred to as "uronic acid polymer degrading enzyme"). In the extrusion process (hereinafter also referred to as "enzymatic extrusion process") high concentrations of uronic acid polymers can be applied. Normally only low concentrations of uronic acid polymers can be processed due to the viscosity increasing properties of these polymers. A high viscosity makes it difficult to mixe the enzymes and the uronic acid polymer. The present extrusion process allows the processing of mixtures with a high concentration of solubilized uronic acid oligosaccharides, while ensuring proper mixing of enzyme and uronic acid polymer.

A main further advantage of the present extrusion process is that it provides an end product with a relatively low water content, thereby reducing drying costs and reducing the formation of undesired products during drying.

The process is particularly suitable for producing uronic acid oligosaccharides with an unsaturated bond in the terminal uronic acid molecule. Unsaturated bonds are chemically more recactive, and hence reduced processing times (including reduced drying time) are desirable for producing these components. Hence the present invention particularly provides an enzymatic extrusion process wherein uronic acid oligosaccharides with preferably double bonds are generated.

In a further embodiment the present invention provides a further process that can be suitably used for the manufacture of uronic acid oligosaccharides (hereinafter also referred to as "high shear extrusion process"). The method comprises a first extrusion step wherein an aqueous uronic acid polymer is reduced in chain length to yield an aqueous composition with partially degraded uronic acid polymer with reduced viscosity. The partially degraded uronic acid polymer is subsequently admixed with an enzyme to further treat the uronic acid molecule, particularly reducing its chain length. Also this process for making uronic acid oligosaccharides including an extrusion step requires reduced quantities of enzyme per uronic acid polymer, and thus is very cost effective. A further advantage is that the present extrusion process results in an end product with a relatively low water content, thereby reducing drying costs and reducing the formation of undesired products during drying.

### DETAILED DESCRIPTION OF THE INVENTION

In one aspect the present invention provides a process for the manufacture of uronic acid oligosaccharides, comprising the steps of:
a. combining at least one uronic acid polymer degrading enzyme, uronic acid polymer and water to obtain a mixture; and
b. extruding the mixture obtained in step a, to obtain a composition containing uronic acid oligosaccharides.

In a further aspect the present invention provides composition, preferably a nutritional or pharmaceutical composition, comprising between 25 and 100 wt.% uronic acid oligosaccharides with a DP between 2 and 250 based on total weight of uronic acid and a dry matter content of between 40 and 60% (weight/weight).

In still a further aspect the present invention provides a process for the manufacture of uronic acid oligosaccharides, comprising the steps of:
a. extruding an aqueous suspension containing uronic acid polymers to obtain a mixture of partially degraded uronic acid polymer; and
b. combining the mixture of partially degraded uronic acid polymer obtained in step a. with a pectin degrading enzyme to obtain a composition containing uronic acid oligosaccharides.

In yet a further embodiment, the present invention provides a process for the manufacture of uronic acid oligosaccharides, comprising extruding an aqueous suspension containing uronic acid polymers which has a pH above 9, to obtain a mixture of uronic acid oligosaccharides or partially degraded uronic acid polymer.

### Uronic acid polymer

The term uronic acid polymer as used in the present invention refers to a polymer wherein preferably at least 50% of the residues are selected from the group consisting of guluronic acid, mannuronic acid, galacturonic acid , iduronic acid, alturonic acid and glucuronic acid, preferably selected from the group consisting of galacturonic acid and glucuronic acid. These may be in free or esterified form. Preferably the uronic acid polymer is selected from the group consisting of pectins, pectates, alginates, chondroitins, hyaluronic acids, heparins, heparans, bacterial carbohydrates and uronic acid-containing carbohydrates.

In a preferred embodiment the uronic acid polymer comprises at least 50 % galacturonic acid based on total uronic acid residues in the polymer, even more preferably the uronic acid polymer comprises at least 50 % galacturonic acid based on total residues in the polymer. More preferably, the present uronic acid polymer is a polygalacturonic acid polymer, preferably pectin, more preferably carrot pectin, apple pectin, citrus pectin or sugar beet pectin. The uronic acid polymer and in particular the galacturonic acid polymer may be methylated and/or amidated. In other words the carboxylic acid group of the (galact)uronic acid unit can be present in the form of an methyl ester or an amide. The uronic acid polymer, preferably pectin, preferably contains methyl esters and/or acetyl esters on the carboxyl group, preferably on the C-2 and/or C-3 atom of the uronic acids. The degree of methylation or acetylation preferably is 20 to 75 %, and in particular 20 to 50 %. Preferably the uronic acid polymer has an average degree of polymerization above 250.

### Enzymes

In the present process, at least one enzyme capable of degrading the uronic acid polymer is used. This enzyme is preferably a lyase, a galacturonase, a esterase or a mixture of these enzymes. Most preferably a lyase is used. More preferably the enzyme used in the present process is selected from the group consisting of hyaluronate lyase, pectate lyase, ply(β-D-mannuronate) lyase, chondroitin ABC lyase, chondroitin AC lyase, oligogalacturonide lyase, heparin lyase heparin sulfate lyase, pectate disaccharide lyase, pectin lyase, poly(α-L-guluronate) lyase, glycuronan lyase, polygalacturonase, β-glucuronidase, hyaluronoglucosaminidase, hyaluronoglucuronidase, glucuronyl-disulfoglucosamine glucuronidase, galacturan 1,4-α galacturonidase, polymannuronidate hydrolyse, pectin esterase and pectin acetylesterase. Most preferably the enzyme is selected from the group consisting of consisting of pectate lyase, oligogalacturonide lyase, pectate disaccharide lyase and pectin lyase.

### Enzymatic extrusion process

In the present process, the uronic acid polymer degrading enzyme, uronic acid polymer and water are combined to obtain a mixture. The term "uronic acid polymer" used herein refers to one uronic acid polymer or to several (two, three ... more ) uronic acid polymers. Preferably the enzyme is added to an aqueous solution comprising the uronic acid polymer to obtain the mixture. The mixture preferably contains at least 10 wt.% uronic acid polymer based on dry weight, more preferably at least 50 wt%, even more preferably at least 75 wt.%. A higher content of uronic acid polymer based on dry matter will yield an oligosaccharide composition of increased purity, which can better be used for further processing. Providing ingredients suitable for further processing, e.g. for inclusion in food products, is a main aim of the present invention.
The mixture preferably contains at least 1 wt.% uronic acid polymer based on total weight of the mixture, more preferably at least 5 wt.%, even more preferably at least 10 wt.%, even more preferably at least 25 wt.%. The uronic acid polymer content preferably does not exceed 90 wt.% based on total weight of the mixture.

Prior to or during extrusion, the enzyme, the uronic acid polymer and water are combined to obtain a mixture. The concentration of enzyme is preferably such that the extrusion process results in an uronic acid oligosaccharide mixture with an average degree of polymerization (DP) between 2 and 150, more preferably with an average DP between 2 and 50 and most preferably a DP between 2-10

The mixture of enzyme and polymer preferably has a pH between 3 and 9, more preferably between 5 and 8. This ensures optimal activity of the enzyme. The temperature of the mixture is preferably between 10°C and 100 °C, more preferably between 25°C and 50°C.

In one embodiment, the present uronic acid oligosaccharides are prepared using co extrusion of a mixture comprising uronic acid polymer, uronic acid polymer degrading enzyme and water. Extrusion preferably takes place in an extruder. The present mixture is preferably extruded at a pressure between 0.1 and 10 bar, even more preferably between about 0.8 and 2 bar.
Preferably the extruder has a single or a twin-screw configuration. The Length/diameter value (L/d) of the extruder is preferably at least 1, more preferably at least 10, even more preferably at least 25. The L/d value preferably does not exceed 1000. A higher L/d value ensures proper mixing and reaction time. The extruder is preferably operated at a screw speed between 10 and 2000 rpm, preferably between 100 and 1000 rpm. Sufficient screw speed ensures a good mixing and interaction between uronic acid polymer and enzyme. The extruder is preferably kept at least partially at a temperature between 10 and 100°C. One additional zone of the extruder (preferably the end zone) preferably has an increased temperature to inactivate the enzymes. Preferably the temperature of this zone is between 90 and 250°C, preferably between 90 and 160 °C.

### High shear extrusion

In a further one embodiment, the present uronic acid oligosaccharides are prepared in a process including a high shear extrusion step, wherein the uronic acid polymers are first broken down to partially degraded uronic acid polymer. In this step the viscosity is reduced, making it more easy have a next processing step wherein the partially degraded uronic acid polymer is further treated, e.g. reduced in chain length.

Hence, the present invention provides a process for the manufacture of uronic acid oligosaccharides, comprising the steps of:
a. extruding an aqueous suspension containing uronic acid polymers to obtain a mixture of partially degraded uronic acid polymer; and
b. combining the mixture of partially degraded uronic acid polymer obtained in step a. with a pectin degrading enzyme to obtain a composition containing uronic acid oligosaccharides.

The viscosity of the mixture of partially degraded uronic acid polymer is preferably below 75 % of the viscosity of the aqueous suspension containing the uronic acid polymer.

In the high shear process, uronic acid polymers and water are combined to obtain an aqueous suspension, preferably an aqueous solution. The suspension preferably contains 10 wt.% uronic acid polymer based on dry weight, more preferably at least 50 wt%, even more preferably at least 75 wt.%. A higher content of uronic acid polymer based on dry matter will yield an oligosaccharide composition or mixture of partially degraded uronic acid polymer of increased purity, which can better be used for further processing. The aqueous suspension preferably contains at least 1 wt.% uronic acid polymer based on total weight of the mixture, more preferably at least 5 wt.%, even more preferably at least 10 wt.%, even more preferably at least 25 wt.%. The uronic acid polymer content of the aqeous suspension preferably does not exceed 90 wt.% based on total weight of the mixture.

For the transformation of uronic acid polymer to partially degraded uronic acid polymer, the uronic acid polymer is preferably subjected to high shear. Preferably the extruder is operated at Specific Mechanical Energy (SME) values of at least 250 kJ/kg, preferably at least 500 kJ/kg. The SME preferably does not exceed 2500 kJ/kg.

Subsequently the partially degraded uronic acid polymer is combined and mixed with an enzyme capable of degrading uronic acid polymer to obtain the present uronic acid oligosaccharide composition.

### Extrusion at pH above 9

In a further one embodiment, the present uronic acid oligosaccharides are prepared in a process including a extrusion step, wherein the uronic acid polymers are broken down to uronic acid oligosaccharides under basic conditions, preferably in a in an aqueous solution with a pH above 9, preferably above 10 even more preferably above 11.

Hence, the present invention also provides a process for the manufacture of uronic acid oligosaccharides, by extruding an aqueous suspension containing uronic acid polymers and having a pH above 9 to obtain a mixture of uronic acid oligosaccharides or partially degraded uronic acid polymer.
In this step the viscosity is reduced, and uronic oligosaccharides or particlly degraded uronic acid polymer has a double bond between the C₄ and C₅ 9see below). One advantage of the present process it that it at least partially removed the need to use enzymes such as lyases.

In the extrusion process at basic conditions, uronic acid polymers and water are combined to obtain an aqueous suspension, preferably an aqueous solution with a pH above 9. The suspension preferably contains 10 wt.% uronic acid polymer based on dry weight, more preferably at least 50 wt%, even more preferably at least 75 wt.%. A higher content of uronic acid polymer based on dry matter will yield an oligosaccharide composition or mixture of partially degraded uronic acid polymer of increased purity, which can better be used for further processing. The aqueous suspension preferably contains at least 1 wt.% uronic acid polymer based on total weight of the mixture, more preferably at least 5 wt.%, even more preferably at least 10 wt.%, even more preferably at least 25 wt.%. The uronic acid polymer content of the aqueous suspension preferably does not exceed 90 wt.% based on total weight of the mixture.

The basic mixture with uronic acid polymer is preferably treated at high pressure, typically at between 1 and 100 bar, even more preferably between 2 and 50 bar. The temperature of the basic mixture with uronic acid polymer during extrusion is preferably above 50°C, even more preferably above 90 °C, even more preferably above 125°C. The temperature is preferably below 250°C.

For the transformation of uronic acid polymer to uronic acid oligosaccharide, the uronic acid polymer is preferably subjected to high shear. Preferably the extruder is operated at Specific Mechanical Energy (SME) values of at least 250 kJ/kg, preferably at least 500 kJ/kg. The SME preferably does not exceed 2500 kJ/kg.

### Uronic acid oligosaccharides

The present processes result in a uronic acid oligosaccharide containing composition. Preferably the present uronic acid oligosaccharide composition obtained or obtainable by the process of the present invention has an average degree of polymerization (DP) between 2 and 250, preferably between 2 and 100, more preferably between 2 and 50, even more preferably between 2 and 10.

In a preferred embodiment, the present uronic acid oligosaccharide composition comprises between 25 and 100 wt.% uronic acid oligosaccharides with a DP between 2 and 250 based on total weight of uronic acid, more preferably between 50 and 100 wt.%, even more preferably between 75 and 100 wt.%. More preferably, the present composition contains between 25 and 100 wt.% uronic acid oligosaccharides with a DP between 2 and 50 based on total weight of uronic acid, more preferably between 50 and 100 wt.%, even more preferably between 75 and 100 wt.%. The uronic acid oligosaccharides are preferably indigestible in the upper intestinal tract of humans and water-soluble.

The present uronic acid oligosaccharide composition preferably comprises between 25 and 100 wt.% uronic acid oligosaccharides with a DP between 2 and 250 based on dry weight of uronic acid oligosaccharide composition, more preferably between 50 and 100 wt.%, even more preferably between 75 and 100 wt.%. More preferably, the present composition contains between 25 and 100 wt.% uronic acid oligosaccharides with a DP between 2 and 50 based on dry weight of uronic acid, more preferably between 50 and 100 wt.%, even more preferably between 75 and 100 wt.%.

Preferably the present uronic acid oligosaccharide composition has a dry matter content of between 20 and 80 % (weight/weight), more preferably between 40 and 60%, even more preferably between 45 and 55 %.

In a preferred embodiment the uronic acid polymer is pectin. Hence, in a preferred embodiment, the present uronic acid oligosaccharide composition comprises between 25 and 100 wt.% galacturonic acid oligosaccharides with a DP between 2 and 250 based on total weight of uronic acid, more preferably between 50 and 100 wt.%, even more preferably between 75 and 100 wt.%. More preferably, the present composition contains between 25 and 100 wt.% galacturonic acid oligosaccharides with a DP between 2 and 50 based on total weight of uronic acid, more preferably between 50 and 100 wt.%, even more preferably between 75 and 100 wt.%. The galacturonic acid oligosaccharides are preferably indigestible in the upper human intestinal tract and water soluble.

In a preferred embodiment, at least one of the terminal uronic units of the uronic acid oligosaccharide has a double bond (i.e. unsaturated uronic acid), which is preferably situated between the C₄ and C₅ position of the terminal hexuronic unit.

Preferably one of the terminal hexuronic units comprises the double bond. The terminal uronic acid preferably has a structure according to the figure below.

Preferred terminal uronic acid group 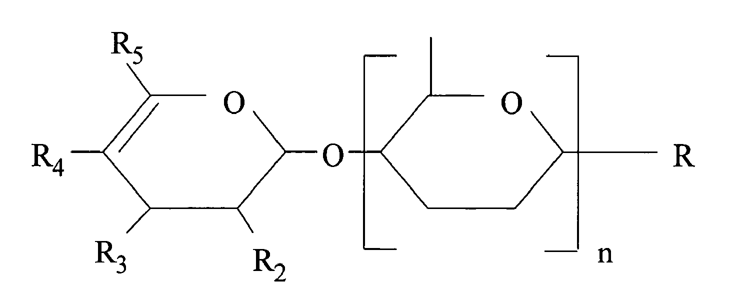 wherein;
R is preferably selected from the group consisting of hydrogen, hydroxyl and acid group, preferably hydroxyl or carboxylic acid group, even more preferably a hydroxyl group (see above); at least one of the radicals R₂, R₃, R₄ and R₅ is selected from the group consisting of free and esterified carboxylic acid, sulfuric acid group and phosphoric acid group, and the remaining radicals R₂, R₃, R₄ and R₅ represent a hydroxyl group and/or hydrogen. Preferably one of the radicals selected from the group consisting of R₂, R₃, R₄ and R₅ represents a free or esterified carboxylic acid, sulfuric acid group or a phosphoric acid group, and the remaining radicals R₂, R₃, R₄ and R₅ represent a hydroxyl group and/or hydrogen. Even more preferably one radical selected from the group consisting of R₂, R₃, R₄ and R₅ represents a free or esterified carboxylic acid and the remaining radicals R₂, R₃, R₄ and R₅ represent a hydroxyl group and/or hydrogen; and n is an integer and refers to a number of uronic acid units, which may be any uronic acid unit. Suitably n is an integer between 1-49 representing the number of uronic acid units said uronic acid units preferably being uronic acid, even more preferably being galacturonic acid units. The carboxylic acid groups on these units may be free or (partly) esterified, and are preferably at least partly methylated.
Most preferably, R₂ and R₃ represent a hydroxyl group, R₄ represent hydrogen and R₅ represents a free or esterified carboxylic acid.

In a further embodiment, the uronic acid oligosaccharides (e.g. pectin oligosaccharides) comprise both unsaturated and saturated terminal hexuronic acid or uronic acid unit. Preferably at least 5%, more preferably at least 10%, even more preferably at least 25% of the terminal uronic acid units of the uronic acid oligosaccharide is an unsaturated hexuronic acid or uronic acid unit. As each individual uronic acid oligosaccharide preferably comprises only one unsaturated terminal uronic acid unit, preferably no more than 50% of the terminal uronic acid units is an unsaturated uronic acid unit (i.e. comprises a double bond).

This aqueous solution of uronic acid oligosaccharides is preferably spray dried, to obtain a powder mixture.

### EXAMPLES

### Example 1: Enzymatic pectin extrusion using a Berstorff ZE25 extruder

GENU pectin USP/100 (Hercules Co., Copenhagen, DK) is dissolved in of 50 mM NaOAc buffer (pH 5.0) is passed through a Berstorff ZE25 twin-screw extruder with a screw diameter of 25 mm and an L/d value of 40. At the entrance pectin powder is added in the screw of the extruder at a feed rate of 10 kg/h. Quickly thereafter water is added at a rate of about 10 kg/h and after an initial mixing period, Pectinase PL by Amano (polygalacturonase) and Rohapect PTE by Röhm (pectin lyase) are added. The extruder was operated at 1 bar and at a controlled temperature of 40°C.
The degree of polymerization of the resulting uronic acid oligosaccharide composition varies and can be influence by adjusting the speed at which the extruder is operated (rotations per minute). The pectin composition obtained by the enzymatic extrusion process has a dry matter content of about 50 wt.% and about 80 wt.% pectin oligosaccharides based on total weight of uronic acid and about 75 wt.% pectin oligosaccharides based on total dry weight of the composition.

## Claims

1. A process for the manufacture of uronic acid oligosaccharides, comprising the steps of:
a. combining at least one uronic acid polymer degrading enzyme, uronic acid polymer and water to obtain a mixture; and
b. extruding the mixture obtained in step a, to obtain an composition containing uronic acid oligosaccharides.

2. A process according to claim 1, wherein the mixture obtained at step a. comprises at least 10 % of uronic acid polymer based on dry weight.

3. A process according to claim 1 or 2, wherein the mixture obtained in step a. contains at least 5 wt.% uronic acid polymer based on total weight of the mixture.

4. A process according to any of the preceding claims, wherein the composition obtained in step b. contains between 25 and 100 wt.% uronic acid oligosaccharides with a DP between 2 and 250 based on the total weight of uronic acid.

5. A process according to any of the preceding claims, wherein the composition obtained in step b. has a dry matter content of between 20 and 80 % (weight/weight).

6. A process according to any of the preceding claims, wherein the temperature of the mixture during the extrusion step b. is adjusted to 10°C to 100 °C.

7. A process according to any of the preceding claims, wherein the uronic acid polymer degrading enzyme is selected from the group consisting of hyaluronate lyase, pectate lyase, ply(β-D-mannuronate) lyase, chondroitin ABC lyase, chondroitin AC lyase, oligogalacturonide lyase, heparin lyase, heparin sulfate lyase, pectate disaccharide lyase, pectin lyase, poly(α-L-guluronate) lyase, glycuronan lyase, polygalacturonase, β-glucuronidase, hyaluronoglucosaminidase, hyaluronoglucuronidase, glucuronyl-disulfoglucosamine glucuronidase, galacturan 1,4-α galacturonidase, polymannuronidate hydrolyse, pectin esterase and pectin acetylesterase.

8. A composition comprising between 25 and 100 wt.% uronic acid oligosaccharides with a DP between 2 and 250 based on total weight of uronic acid and a dry matter content of between 40 and 60% (weight/weight).

9. A process for the manufacture of uronic acid oligosaccharides, comprising the steps of:
a. extruding an aqueous suspension containing uronic acid polymers to obtain a mixture of partially degraded uronic acid polymer; and
b. combining the mixture of partially degraded uronic acid polymer obtained in step a with a pectin degrading enzyme to obtain a composition containing uronic acid oligosaccharides.

10. A process according to claim 9, wherein the suspension solution containing uronic acid polymers is extruded at Specific Mechanical Energy (SME) values of at least 250 kJ/kg.

11. A process for the manufacture of uronic acid oligosaccharides, comprising extruding an aqueous suspension containing uronic acid polymers which has a pH above 9, to obtain a mixture of uronic acid oligosaccharides or partially degraded uronic acid polymer.
